# EUROPEAN PATENT APPLICATION

(11) **EP 3 308 706 A1**
(43) Date of publication of application: **18.04.2018**
(21) Application number: 16807744.4
(22) Date of filing: 03.06.2016
(51) Int. Cl.: A61B 5/145, A61B 5/00, G01N 27/327, G01N 27/48, G01N 33/53, G01N 33/66, H01L 51/00, G01N 27/30

(54) **BIOSENSOR AND METHOD FOR FORMING SAME AND GLUCOSE CONTROL SYSTEM, METHOD FOR FORMING THE GLUCOSE CONTROL SYSTEM, AND METHOD FOR CONTROLLING GLUCOSE THEREBY**

(30) Priority: 12.06.2015 KR 20150083611; 12.06.2015 KR 20150083637; 21.03.2016 KR 20160033648; 21.03.2016 KR 20160033652
(71) Applicant: Seoul National University R&DB Foundation, Seoul 08826 (KR); Institute for Basic Science, Daejeon 34047 (KR)
(72) Inventor: KIM, Daehyeong, Incheon 21986 (KR); HYEON, Taeghwan, Seoul 06277 (KR); CHOI, Seunghong, Seoul 07995 (KR); LEE, Hyunjae, Incheon 21594 (KR); CHOI, Taekyu, Chuncheon-si Gangwon-do 24414 (KR)
(74) Representative: Ter Meer Steinmeister & Partner
(86) International application number: PCT/KR2016/005949
(87) International publication number: WO 2016/200104

(57) **Abstract**

Provided are a biosensor and a method for forming the same, a glucose control system, a method for forming the glucose control system, and a method for controlling glucose using the glucose control system. The biosensor comprises at least one sensor comprising a sensing unit, a bridge unit connected to the sensing unit, and an electrode unit connected to the bridge unit, wherein the sensing unit comprises a graphene layer. The method for forming the biosensor that comprises at least one sensor comprising a sensing unit, a bridge unit connected to the sensing unit and an electrode unit connected to the bridge unit, comprises forming a lower insulation layer, forming a conductive electrode layer on the lower insulation layer, forming a graphene layer on the conductive electrode layer and forming a reaction layer on the graphene layer.

The glucose control system comprises a sensor unit comprising a glucose sensor, a glucose regulation unit regulating glucose concentration in a body of a user and a control unit controlling the sensor unit and the glucose regulation unit. The method for forming a glucose control system comprises forming a sensor unit comprising a glucose sensor, forming a glucose regulation unit and packaging the sensor unit and the glucose regulation unit.

## Description

### Technical Field

The present invention relates to a biosensor and a method for forming the same, a glucose control system, a method for forming the glucose control system, and a method for controlling glucose using the glucose control system.

### Background Art

An electrochemical based biosensor is to combine the analytical ability that an electrochemical method has and the specificity of biological recognition. That is, biological recognition phenomena can be detected as the change of electric current or potential by holding or including a biospecific reagent such as enzyme, antigen, antibody, biochemical substance and the like at the surface of an electrode. In this electrochemical based biosensor, the resistance of the electrode itself and the feature of the interface where an electrochemical reaction occurs are very important.

Graphene is attracting attention as one of the nanomaterials capable of manufacturing an electrochemical based biosensor with improved performance. However, since graphene is chemically inert, it is difficult to make use of graphene to realize an electrochemical based biosensor. Therefore, the surface activation of graphene is needed in order to manufacture an electrochemical based biosensor using graphene. In addition, since graphene oxide was mainly used in conventional biosensors using graphene and the graphene oxide is based on a solution process, there was a difficulty in the manufacturing process.

The above mentioned example is disclosed in Korean Patent No.10-1355933 (Title: Method for adsorption of various biomaterials on chemically modified graphene). Concretely, in order to adsorb a hydrophilic biomaterial on graphene that basically has a hydrophilic property, a modification process where reduction and nitrogen doping are carried out at the same time is needed. Herein, the reduction is for restoring the electrical properties of graphene oxide produced by oxidizing the graphite, and the nitrogen doping is for adsorbing the hydrophilic biomaterial. Since the biomaterial is selectively adsorbed only on the modified graphene through the modification process, the Korean Patent provides a method for producing a composite substrate comprising a patterned graphene layer that selectively adsorbs the biomaterial.

Meanwhile, a diabetes prevalence rate is increasing due to aging society and westernized lifestyle. Diabetes can lead to complications in major organs of the body if there is no proper blood sugar control in the long term. Therefore, it is very important to maintain blood sugar normally.

Accurate blood sugar measurement is very important for proper blood sugar control. However, most of conventional blood sugar monitoring devices have a disadvantage of causing pain and inconvenience to patients because the blood sugar is measured by taking blood in an invasive manner. Therefore, the development of a noninvasive blood sugar monitoring device capable of measuring the blood sugar without drawing blood is under study.

In addition, the importance of blood sugar monitoring devices is growing bigger and bigger because hypoglycemia resulting from insulin treatment frequently occurs in diabetic patients. Therefore, it is necessary to develop a measurement and control device for blood sugar that is capable of not only measuring blood sugar concentration accurately but also controlling blood sugar simultaneously.

In this regard, Korean Patent No.10-0553801 (Title: The closed loop type realtime insulin pump by using of skin contacted glucose detection sensor in blood) provides a real-time insulin pump that is a close loop type and uses a blood sugar detection sensor of skin contact type. According to the real-time insulin pump, it is safe and accurate by using the blood sugar detection sensor of skin contact type, the blood sugar can be detected in real time, the measurement of blood sugar and the injection of insulin can be controlled in real time by applying an insulin pump controlling a precise insulin injection in open loop type, and thus it is possible to regulate the most accurate blood sugar level for patients.

### Disclosure

### Technical Problem

In order to solve the above mentioned problems, the present invention provides a biosensor exhibiting excellent reliability.

The present invention provides a biosensor exhibiting excellent flexibility and stretchability.

The present invention provides a biosensor including various sensors on one platform.

The present invention provides a method for forming the biosensor.

The present invention provides a glucose control system that can measure glucose concentration in a noninvasive manner.

The present invention provides a glucose control system that can control glucose concentration in a body of a user.

The present invention provides a glucose control system exhibiting excellent flexibility and stretchability.

The present invention provides a method for forming the glucose control system.

The present invention provides a method for controlling glucose using the glucose control system.

The other objects of the present invention will be clearly understood by reference to the following detailed description and the accompanying drawings.

### Technical Solution

A biosensor according to embodiments of the present invention comprises at least one sensor comprising a sensing unit, a bridge unit connected to the sensing unit and an electrode unit connected to the bridge unit, and the sensing unit comprises a graphene layer.

The graphene layer may comprise a doped conductive substance. The conductive substance may comprise at least one selected from metal nanoparticles and metal nanowires.

The sensing unit may further comprise a conductive electrode layer disposed under the graphene layer and a reaction layer disposed on the graphene layer. The conductive electrode layer may comprise net patterns or mesh patterns.

The sensing unit may further comprise an upper insulation layer disposed on the graphene layer and the upper insulation layer may have an opening part exposing the graphene layer. The reaction layer may be in contact with the graphene layer through the opening part.

The reaction layer may be formed of different substances depending on a type of the sensor. The reaction layer may be formed of silver/silver chloride or PEDOT. A surface of the reaction layer may be treated by at least one substance selected from polyaniline, prussian blue and glucose oxidase.

The bridge unit may have a multiply-bent shape.

The sensor may comprise at least one sensor selected from a humidity sensor, a pH sensor, a glucose sensor and a strain gauge.

The biosensor may further comprise an electric source unit providing an electric source to the electrode unit and a processing unit that receives any one signal of electric current, voltage or impedance from the electrode unit to convert the signal.

In a method for forming a biosensor according to embodiments of the present invention, the biosensor comprises at least one sensor comprising a sensing unit, a bridge unit connected to the sensing unit and an electrode unit connected to the bridge unit, and the method comprises forming a lower insulation layer, forming a conductive electrode layer on the lower insulation layer, forming a graphene layer on the conductive electrode layer and forming a reaction layer on the graphene layer.

The method for forming a biosensor may further comprise forming an upper insulation layer on the graphene layer wherein the upper insulation layer has an opening part that exposes the graphene layer, and doping a conductive substance at the graphene layer through the opening part before forming the reaction layer.

The conductive substance may comprise at least one selected from metal nanoparticles and metal nanowires.

The reaction layer may be formed on the graphene layer through the opening part.

The conductive electrode layer may comprise net patterns or mesh patterns. The conductive electrode layer may be formed of a substance comprising at least one selected from gold, aluminum, platinum, nickel, graphene, silver nanowire film, metal grid and indium tin oxide.

The reaction layer may be formed of different substances depending on a type of the sensor.

The sensing unit, the bridge unit and the electrode unit may be formed together by a same process.

The sensor may comprise at least one sensor selected from a humidity sensor, a pH sensor, a glucose sensor and a strain gauge.

A glucose control system according to embodiments of the present invention comprises a sensor unit comprising a glucose sensor, a glucose regulation unit regulating glucose concentration in a body of a user and a control unit controlling the sensor unit and the glucose regulation unit.

The sensor unit may further comprise at least one selected from a humidity sensor, a pH sensor and a strain gauge.

The control unit may receive signals from the glucose sensor to measure glucose concentration in sweat of the user, receive signals from the pH sensor to measure pH value, and amend the measured glucose concentration based on the pH value.

The control unit may receive signals from the humidity sensor to measure humidity, receive signals from the strain gauge to measure strain, and amend the measured glucose concentration based on the pH value, the humidity and the strain.

The control unit may receive signals from the humidity sensor to measure humidity, and receive signals from the glucose sensor to measure the glucose concentration in sweat of the user when the humidity is equal to or more than a predetermined value.

The control unit may diagnose a blood sugar state in the body of the user based on the amended glucose concentration. The control unit may control the glucose regulation unit in order to inject a glucose regulation drug into the user based on the diagnosed blood sugar state in the body of the user.

The control unit may receive signals from the strain gauge to measure strain, and diagnose a blood sugar state in the body of the user as a low blood sugar state based on the strain.

The glucose regulation unit may comprise a drug delivery part that comprises a fine needle comprising a glucose regulation drug and a heating part that is disposed on the drug delivery part and heats the drug delivery part to increase a temperature of the drug delivery part, a surface of the drug delivery part may be coated with a phase change material whose phase is changed at critical temperature or more, and the glucose regulation drug may be released from the fine needle by heating the heating part.

The drug delivery part may inject the glucose regulation drug through the user's skin by the fine needle, and the glucose regulation drug may comprise blood sugar depressing agents.

The heating part may comprise a first heating part, a second heating part adjacent to the first heating part, and a temperature sensor that is disposed between the first and second heating parts and measures temperature of the first and second heating parts. The control unit may control temperature of the first and second heating parts when the temperature measured by the temperature sensor is equal to or more than a predetermined temperature. The heating part may comprise multiply-bent patterns.

The sensor unit may comprise a sensing unit, a bridge unit connected to the sensing unit and an electrode unit connected to the bridge unit, and the sensing unit may comprise a graphene layer.

The graphene layer may comprise a doped conductive substance, and the conductive substance may comprise at least one selected from metal nanoparticles and metal nanowires.

The sensing unit may further comprise a conductive electrode layer disposed under the graphene layer and a reaction layer disposed on the graphene layer, the conductive electrode layer may comprise net patterns or mesh patterns, and the reaction layer may be formed of different substances depending on a type of the sensing unit.

The bridge unit may have a multiply-bent shape.

The glucose control system may further comprise a network transmission and reception unit. The network transmission and reception unit may send the blood sugar state in the body of the user diagnosed by the control unit to a user terminal interacting with the glucose control system.

A method for forming a glucose control system according to embodiments of the present invention comprises forming a sensor unit comprising a glucose sensor, forming a glucose regulation unit and packaging the sensor unit and the glucose regulation unit.

The step of forming a glucose regulation unit may comprise forming a heating part, forming a drug delivery part that comprises a fine needle comprising a glucose drug, combining the heating part and the drug delivery part, and coating the surface of the drug delivery part with a phase change material.

The sensor unit may comprise at least one sensor comprising a sensing unit, a bridge unit connected to the sensing unit and an electrode unit connected to the bridge unit, and the step of forming the sensor unit may comprise forming a lower insulation layer, forming a conductive electrode layer on the lower insulation layer, forming a graphene layer on the conductive electrode layer, and forming a reaction layer on the graphene layer.

The method for forming a glucose control system may further comprise forming an upper insulation layer on the graphene layer wherein the upper insulation layer has an opening part that exposes the graphene layer, and doping a conductive substance at the graphene layer through the opening part before forming the reaction layer wherein the conductive substance comprises at least one selected from metal nanoparticles and metal nanowires.

The reaction layer may be formed on the graphene layer through the opening part, and the reaction layer may be formed of different substances depending on a type of the sensor.

A method for controlling glucose according to embodiments of the present invention, uses a glucose control system that comprises a sensor unit comprising a glucose sensor and a pH sensor, a glucose regulation unit regulating glucose concentration in a body of a user, and a control unit controlling the sensor unit and the glucose regulation unit, and comprises receiving signals from the glucose sensor to measure glucose concentration in sweat of the user, receiving signals from the pH sensor to measure pH value and amending the measured glucose concentration based on the pH value.

The sensor unit may further comprise at least one selected from a humidity sensor and a strain gauge, the method for controlling glucose may further comprise receiving signals from the humidity sensor to measure humidity and receiving signals from the strain gauge to measure strain, and the measured glucose concentration may be amended based on the pH value, the humidity and the strain.

The method for controlling glucose may further comprise diagnosing a blood sugar state in the body of the user based on the amended glucose concentration.

The method for controlling glucose may further comprise injecting a glucose regulation drug into the user by the glucose regulation unit based on the diagnosed blood sugar state in the body of the user.

The sensor unit may further comprise a humidity sensor, the method for controlling glucose may further comprise receiving signals from the humidity sensor to measure humidity, and signals may be received from the glucose sensor and the glucose concentration in sweat of the user may be measured when the humidity is equal to or more than a predetermined value.

The method for controlling glucose may further comprise receiving signals from the strain gauge to measure strain, and a blood sugar state in the body of the user may be diagnosed as a low blood sugar state based on the strain.

### Advantageous Effects

A biosensor according to embodiments of the present invention can have excellent reliability since various features such as the feature of the interface are improved. The biosensor can be easily and variously applied to a wearable apparatus that is used in the state of being attached to the body since it can have excellent flexibility and stretchability. The biosensor can include various sensors on one platform so that various substances can be detected simultaneously. In the biosensor, it is not necessary to prepare a reference electrode separately since reference electrode and working electrode can be formed together in one platform. The biosensor may include one or more sensors, and the one or more sensors may be easily formed by a simple process.

The glucose control system according to embodiments of the present invention can measure glucose concentration in a noninvasive manner. The glucose control system can accurately measure glucose concentration in the user's body by amending the measured glucose concentration with reference to pH value, humidity, strain, etc. The glucose control system can control the glucose concentration in the user's body while measuring the glucose concentration in real time. The glucose control system can have excellent flexibility and stretchability so that it can be easily used in the state of being attached to a body.

### Description of Drawings

Fig. 1 is a schematic view of a biosensor according to an embodiment of the present invention.
Fig. 2 shows the constitution of a biosensor according to an embodiment of the present invention.
Fig. 3 is a view for explaining the constitution of a biosensor according to an embodiment of the present invention.
Fig. 4 is a view for explaining a sensing unit of a biosensor according to an embodiment of the present invention.
Fig. 5 is an enlarged view for a conductive electrode layer formed on the upper part of a lower insulation layer of a bridge unit of a biosensor according to the present invention.
Fig. 6 is a flowchart for explaining a method of forming a biosensor according to an embodiment of the present invention.
Fig. 7 is a view for explaining a method of forming a biosensor according to an embodiment of the present invention.
Fig. 8 schematically shows a process of forming a biosensor according to an embodiment of the present invention.
Fig. 9 is a view for explaining a process of treating the surface of a biosensor according to an embodiment of the present invention.
Fig. 10 is an image of a biosensor formed according to a embodiment of the present invention.
Fig. 11 shows deposition results of polyethylenedioxythiophene depending on electrode structures composing a biosensor according to an embodiment of the present invention.
Fig. 12 is graphs analyzing the feature of electrodes related to deposition results of polyethylenedioxythiophene depending on electrode structures composing a biosensor according to an embodiment of the present invention.
Fig. 13 is graphs analyzing the electrochemical feature depending on electrode structures composing a biosensor according to an embodiment of the present invention.
Fig. 14 is graphs analyzing operational features of a biosensor according to an embodiment of the present invention.
Fig. 15 shows the stretchability of a biosensor according to an embodiment of the present invention.
Fig. 16 shows the constitution of a glucose control system according to an embodiment of the present invention.
Fig. 17 shows a regulation unit of a glucose control system according to an embodiment of the present invention.
Fig. 18 is an image of a heating part according to an embodiment of the present invention.
Fig. 19 is a sectional view of a heating part of a glucose control system according to an embodiment of the present invention.
Fig. 20 shows a drug delivery part in a glucose control system according to an embodiment of the present invention.
Fig. 21 shows a fine needle of a drug delivery part in a glucose control system according to an embodiment of the present invention.
Fig. 22 is a flowchart for explaining a method for forming a glucose control system according to an embodiment of the present invention.
Fig. 23 is a flowchart for explaining a method for forming a glucose regulation unit according to an embodiment of the present invention.
Fig. 24 is a flowchart for explaining a method for forming a heating part of a glucose regulation unit according to an embodiment of the present invention
Fig. 25 is a view for explaining a formation of a drug delivery part and a method of combining a drug delivery part and a heating part according to an embodiment of the present invention.
Fig. 26 is an image of a sensor unit and a glucose regulation unit formed according to an embodiment of the present invention.
Fig. 27 is an image of a glucose control system formed according to an embodiment of the present invention.
Fig. 28 is a flowchart for explaining a method for controlling glucose using a glucose control system according to an embodiment of the present invention.
Fig. 29 is a view for explaining an operation of a sensor unit in a glucose control system according to an embodiment of the present invention.
Fig. 30 is a view for explaining an operation of a glucose regulation unit in a glucose control system according to an embodiment of the present invention.

### Best Mode

Hereinafter, a detailed description will be given of the present invention with reference to the following embodiments. The purposes, features, and advantages of the present invention will be easily understood through the following embodiments. The present invention is not limited to such embodiments, but may be modified in other forms. The embodiments to be described below are nothing but the ones provided to bring the disclosure of the present invention to perfection and assist those skilled in the art to completely understand the present invention. Therefore, the following embodiments are not to be construed as limiting the present invention.

It will be understood that, although the terms first, second, third etc. may be used herein to describe various elements, components, regions, layers and/or sections, these elements, components, regions, layers and/or sections should not be limited by these terms. These terms are only used to distinguish one element, component, region, layer or section from another element, component, region, layer or section. Thus, a first element, component, region, layer or section discussed below could be termed a second element, component, region, layer or section without departing from the teachings of the present invention.

It will be understood that when an element is referred to as being "on" another element, it can be directly on the other element or intervening elements may be present therebetween. In contrast, when an element is referred to as being "directly on" another element, there are no intervening elements present.

The size of the element or the relative sizes between elements in the drawings may be shown to be exaggerated for more clear understanding of the present invention. In addition, the shape of the elements shown in the drawings may be somewhat changed by variation of the manufacturing process or the like. Accordingly, the embodiments disclosed herein are not to be limited to the shapes shown in the drawings unless otherwise stated, and it is to be understood to include a certain amount of variation.

It will be understood that when an element is referred to as being "connected" with another element, the element can be directly connected with another element or they can be connected with intervening elements present therebetween. In addition, when an element is referred to as "including" another element, it means that the element can include further element(s) as well as another element unless specifically stated to the contrary.

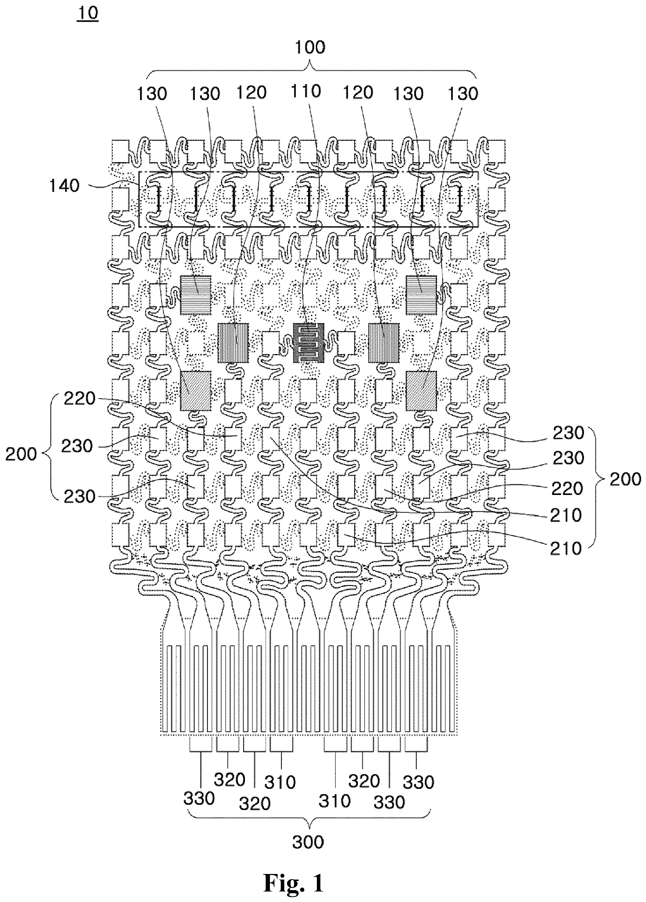

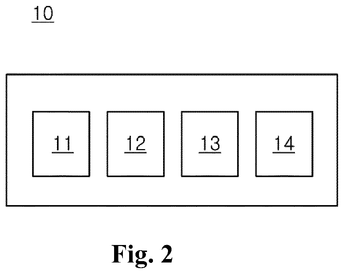

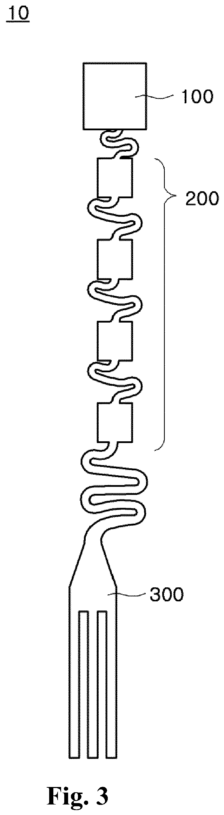

Fig. 1 is a schematic view of a biosensor according to an embodiment of the present invention, Fig. 2 shows the constitution of a biosensor according to an embodiment of the present invention, and Fig. 3 is a view for explaining the constitution of a biosensor according to an embodiment of the present invention.

Referring to Figs. 1 to 3, a biosensor 10 includes a sensing unit 100, a bridge unit 200 and an electrode unit 300.

The sensing unit 100 may include a plurality of sensing units disposed in arbitrary patterns. The sensing unit 100 may include a first sensing unit 110, a second sensing unit 120, a third sensing unit 130 and a forth sensing unit 140. The first sensing unit 110 may be a humidity sensing unit that can measure the humidity, the second sensing unit 120 may be a pH sensing unit that can measure the pH, the third sensing unit 130 may be a glucose sensing unit that can measure the glucose, and the forth sensing unit 140 may be a strain gauge that can measure the strain. One of each or two or more of the first sensing unit 110, the second sensing unit 120, the third sensing unit 130 and the forth sensing unit 140 may be disposed.

The biosensor 10 may include a first sensor 11, a second sensor 12, a third sensor 13 and a forth sensor 14. The first sensor 11 may be a humidity sensor, the second sensor 12 may be a pH sensor, the third sensor 13 may be a glucose sensor, and the forth sensor 14 may be a strain gauge. The first sensor 11 may include the first sensing unit 110, a first bridge unit 210 and a first electrode unit 310. The second sensor 12 may include the second sensing unit 120, a second bridge unit 220 and a second electrode unit 320. The third sensor 13 may include the third sensing unit 130, a third bridge unit 230 and a third electrode unit 330. The forth sensor 14 may include the forth sensing unit 140, a forth bridge unit 240 and a forth electrode unit 340.

The bridge unit 200 connects the sensing unit 100 and the electrode unit. The electrode unit 300 can provide voltage or receive signals such as measured potential difference, electric current, impedance and the like from the sensing unit 100. The electrode unit 300 may include two or three electrode patterns in order to receive the signals.

Even though not shown in drawings, the biosensor 10 may further include an electric source unit that provides the electric source and a processing unit that converts the signals such as potential difference, electric current, impedance and the like collected by the electrode unit 300 to the ion change amount or the concentration change amount of chemical substances by oxidation-reduction reaction. The electric source unit can provide the electric power to the electrode unit 300 using an electric power cable, a rechargeable battery or a disposable battery.

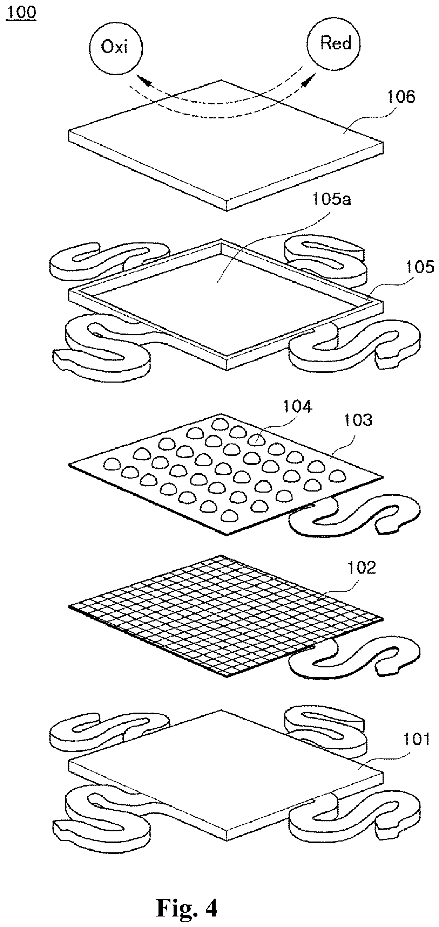

Fig. 4 is a view for explaining a sensing unit of a biosensor according to an embodiment of the present invention.

Referring to Fig. 4, the sensing unit 100 may include a lower insulation layer 101, a conductive electrode layer 102, a graphene layer 103, an upper insulation layer 105 and a reaction layer 106.

The lower insulation layer 101 may be formed of a high molecular substance that is transparent and nonconductive. For example, the high molecular substance may be photosensitive polymer. In addition, for example, the high molecular substance may be epoxy resin, polyimide, parylene.

The conductive electrode layer 102 is disposed on the lower insulation layer 101. The external shape of the conductive electrode layer 102 may be identical to the lower insulation layer 101, but its internal shape may include net patterns or mesh patterns. The conductive electrode layer 102 may be formed of a conductive substance where the electrical current can flow. The conductive substance may include at least one selected from metal such as Au, Al, Pt, Ni, graphene, siver nanowire film, metal grid and metal oxide such as indium tin oxide (ITO).

The graphene layer 103 is disposed on the conductive electrode layer 102. The graphene layer 103 may include graphene, carbonaceous material such as graphene, carbon nanotube (CNT), etc. Conductive substances 104 may be doped in the graphene layer 103. The conductive substances 104 may be disposed on the entire region or some areas of the graphene layer 103 in the form of metal nanoparticles or metal nanowires. For example, the conductive substances 104 may be include Au nanoparticles, and the conductivity of the graphene layer 103 can be improved by the doped conductive substances 104.

The upper insulation layer 105 is disposed on the graphene layer 103. The upper insulation layer 105 may have an opening part 105a that expose some portion of the graphene layer 103.

The reaction layer 106 is disposed on the graphene layer 103 exposed by the opening part 105a. The reaction layer 106 may be formed by depositing Ag/AgCl or Poly(3,4-ethylenedioxythiophene) (PEDOT) in order to be used as a reference electrode of the glucose sensor or measure the humidity.

At least one reaction layer of the reaction layers 106 may be formed by depositing a substance for forming a counter electrode. Therefore, in an electrochemical based biosensor according to another embodiment of the present invention, a plurality of sensing units 100 are formed on one platform and the reaction layer 106 of each sensing unit 100 detects a substance different from a substance detected by another reaction layer so that various substances can be detected. In addition, a separate commercialized reference electrode should be prepared in the conventional electrochemical sensor, but reference electrode and working electrode can be formed simultaneously in one platform in the present invention.

The surface of the reaction layer 106 may be treated by polyaniline in order to measure acidity (pH). In addition, the surface of the reaction layer 106 may be treated by glucose oxidase, prussian blue and the like in order to measure glucose in sweat. Herein, the glucose oxidase is a breakdown enzyme of glucose, and the prussian blue functions as a catalyst in the breakdown of peroxide that is the product of the breakdown of glucose. Substances for the reaction layer 106 may be different from each other depending on an object to be detected by the sensing unit 100.

The sensing unit 100 may include the reaction layer 106 for detecting different substances on one platform, and thus a plurality of sensors 11 to 14 detecting various substances can be realized. In addition, a separate commercialized reference electrode is not needed by forming reference electrode and working electrode together in one platform.

Even though not shown in drawings, the bridge unit 200 may include a lower insulation layer, a conductive electrode layer, a graphene layer and an upper insulation layer. The electrode unit 300 may include a lower insulation layer and a conductive electrode layer. The electrode unit 300 may include an upper insulation layer on the conductive electrode, and the upper insulation layer includes an opening part like the upper insulation layer 105 of the sensing unit 100. The electric source of the electric source unit can be provided to the electrode unit 300 through the opening part. The lower insulation layer, the conductive electrode layer, the graphene layer and the upper insulation layer of the bridge unit 200 may be formed of the same material and by the same process as the lower insulation layer 101, the conductive electrode layer 102, the graphene layer 103 and the upper insulation layer 105 of the sensing unit 100, respectively. It does not matter even if a conductive substance is not doped on the graphene layer of the bridge unit 200.

The biosensor 10 includes a plurality of sensing units 100 in one platform so that each sensing unit 100 can detects a substance different from a substance detected by another sensing unit. The lower insulation layer composing the sensing unit 100, the bridge unit 200 and the electrode unit 300 may be interconnected, even though the interconnected lower insulation layer belongs to different sensors. However, the conductive electrode layer composing the sensing unit 100, the bridge unit 200 and the electrode unit 300 may be electrically divided per each sensor in order that the sensing unit, the bridge unit and the electrode unit are connected to operate as one sensor. The sensing unit, the bridge unit and the electrode unit can be connected to operate as one sensor and receive signals individually.

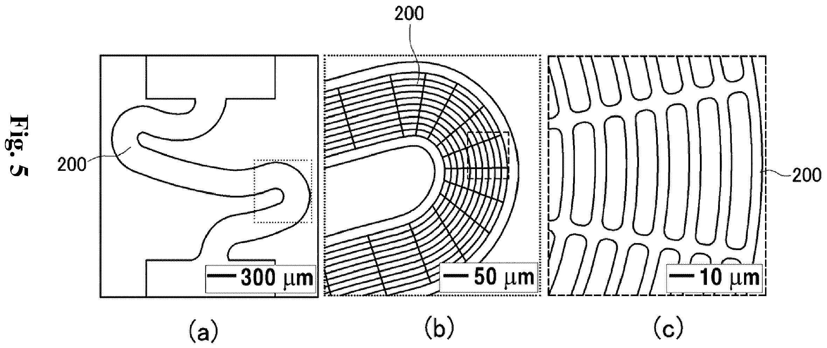

Fig. 5 is an enlarged view for a conductive electrode layer formed on the upper part of a lower insulation layer of a bridge unit of a biosensor according to the present invention.

Referring to Fig. 5, the lower insulation layer belonging to the bridge unit 200 may include patterns multiply bent in the up/down or left/right direction. In addition, the conductive electrode layer disposed on the lower insulation layer of the bridge unit 200 may be formed in net patterns or mesh patterns along the shape of the multiply-bent lower insulation layer.

Since the bridge unit 200 connecting the sensing unit 100 and the electrode unit 300 is formed in multiply-bent patterns, the biosensor can have stretchability. As a result, it does not cause any inconvenience to users when being worn by them so that it can be easily applied to a wearable apparatus that is used in the state of being worn in the body.

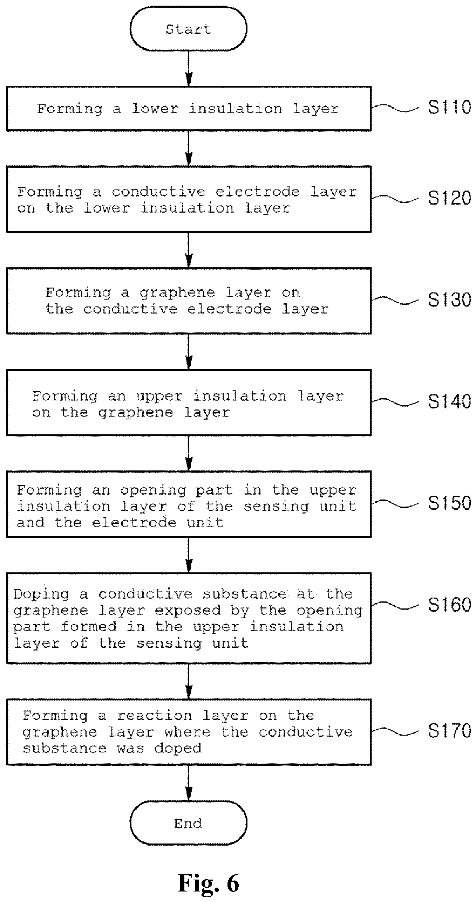

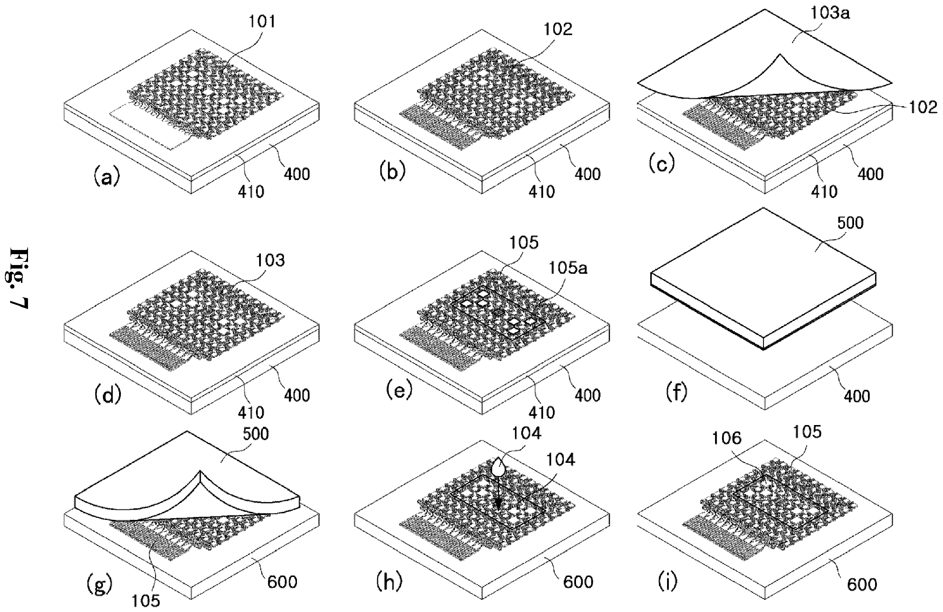

Fig. 6 is a flowchart for explaining a method of forming a biosensor according to an embodiment of the present invention, and Fig. 7 is a view for explaining a method of forming a biosensor according to an embodiment of the present invention.

Referring to Figs. 6 and 7, a method for forming a biosensor may include step S110 of forming a lower insulation layer 101, step S120 of forming a conductive electrode layer 102 on the lower insulation layer 101, step S130 of forming a graphene layer 103 on the conductive electrode layer 102, step S140 of forming an upper insulation layer 105 on the graphene layer 103, step S150 of forming an opening part 105a in the upper insulation layer 105 of the sensing unit and the electrode unit, step S160 of doping a conductive substance 104 at the graphene layer 103 exposed by the opening part 105a formed at the sensing unit, and step S170 of forming a reaction layer 106 on the graphene layer 103 where the conductive substance was doped.

In step S110, the lower insulation layer 101 is formed on a sacrifice substrate 400. Glass substrate, quartz substrate, silicone substrate, germanium substrate and the like may be used as the sacrifice substrate. A sacrifice layer 410 for separating the biosensor from the substrate may be formed before forming the lower insulation layer 101 on the sacrifice substrate 400. For example, the sacrifice layer 410 is formed of metals such as Ni, Cu, Al or PMMA (poly(methyl methacrylate), etc.

The lower insulation layer 101 can be formed by coating photosensitive polymer substance on the sacrifice substrate 400 and carrying out photolithography or e-beam lithography process. The polymer substance may include at least one selected from epoxy resin, polyimide and parylene.

The lower insulation layer 101 disposed at the sensing unit 100, the lower insulation layer 101 disposed at the bridge unit 200 and the lower insulation layer 101 disposed at the electrode unit 300 may be formed in different patterns. However, in order to facilitate the fabrication process, it is possible to pattern the lower insulation layer 101 belonging to the sensing unit 100, the bridge unit 200 and the electrode unit 300 so as to be interconnected.

In step S120, the conductive electrode layer 102 is formed on the lower insulation layer 101. The conductive electrode layer 102 can be formed by depositing a conductive substance on the lower insulation layer 101 and carrying out photolithography or e-beam lithography. The conductive substance may include at least one selected from metals such as Au, Al, Pt, Ni and the like, graphene, silver nanowire film, metal grid and metal oxide such as indium tin oxide (ITO), etc.

Each conductive electrode layer 102 belonging to a plurality of the sensing unit 100, the bridge unit 200 and the electrode unit 300 can be formed together through a single process. The conductive electrode layer 102 formed in the sensing unit 100 and the bridge unit 200 may be formed in net patterns or mesh patterns. The patterns of each conductive electrode layer 102 belonging to the sensing unit 100, the bridge unit 200 and the electrode unit 300 that compose one sensor are interconnected. However, the conductive electrode layer 102 can make each sensor electrically insulated.

Even though the lower insulation layer 101 belonging to a plurality of sensors is interconnected, each sensor can independently measure and receive signals since each sensor is electrically divided by the conductive electrode layer 102.

In step S130, the graphene layer 103 is formed on the conductive electrode layer 102. Graphene 103a may be grown by chemical vapor deposition (CVD) to be transferred onto the conductive electrode layer 102. The graphene 103a may be formed in a single layer or in two or more layers, and may be grown directly on the conductive electrode layer 102. The graphene layer 103 can be formed on the sensing unit 100 and the bridge unit 200 by patterning the graphene 103a in the same shape as the conductive electrode layer 102 except for the electrode unit 300.

In step S140, the upper insulation layer 105 is formed on the graphene layer 103. The upper insulation layer 105 can be formed by coating a nonconductive photosensitive high molecular substance on the graphene layer 103 of the sensing unit 100 and the bridge unit 200 and on the conductive electrode layer 102 of the electrode unit 300 and patterning the coated substance in the same shape as the lower insulation layer 101. Since the method for forming the upper insulation layer 105 is the same as the method for forming the lower insulation layer 101 that was previously explained, the detailed explanation is not made here.

In step S150, the opening part is formed in the upper insulation layer of the sensing unit 100 and the electrode unit 300. The upper insulation layer 105 of the sensing unit 100 and the electrode unit 300 can be removed by using a photolithography or e-beam lithography process. The upper insulation layer 105 existing on the sensing unit 100 and the electrode unit 300 is removed, and the graphene layer 103 of the sensing unit 100 and the conductive electrode layer 102 of the electrode unit 300 can be exposed through the opening part 105a.

Step 140 of forming the upper insulation layer 105 and step 150 of forming the opening part 105a in the upper insulation layer 105 of the sensing unit 100 and the electrode unit 300 can be carried out simultaneously in a single process using one mask.

In step S160, the conductive substance 104 is doped at the graphene layer 103 exposed by the opening part 105a formed in the upper insulation layer 105. The conductive substance 104 may include at least one selected from metal nanoparticles and metal nanowires. For example, the conductive substance 104 may include gold nanoparticles. For example, gold nanoparticles can be doped in the graphene layer 103 by carrying out a drop casting of a gold chloride solution to the exposed graphene layer 103.

In step S170, the reaction layer 106 is formed on the graphene layer 103 where the conductive substance is doped. The reaction layer 106 capable of detecting a specific substance can be formed in the graphene layer exposed by the opening part 105a.

Even though not shown in drawings, the method of forming the biosensor may further include a step of treating a surface of the reaction layer 106 with organic molecules that selectively react with a specific substance. For example, the surface of the reaction layer 106 may be treated with materials suchs as polyaniline, glucose oxidase, prussian blue, etc.

In order to realize various sensors in one platform, the reaction layer 106 belonging to the sensing unit 100 can be formed of different substances depending on the sensor or the surface of the reaction layer 106 can be treated with different substances.

The reaction layer 106 can be selectively formed by providing an electronic source to the electrode unit 300 connected with the sensing unit 100 where the reaction layer 106 will be formed to flow electric currents through the conductive electrode layer 102 of the sensing unit 100. By this, each reaction layer 106 belonging to the sensing unit 100 can be formed of different substances depending on the sensor. That is, by providing the electrical source to the electrode unit 300 connected with the sensing unit 100 where the reaction layer 106 will be formed, the reaction layer 106 can be selectively formed on only a specific graphene layer 103 of a plurality of graphene layers 103 exposed by the opening part 105a. Herein, the specific graphene layer 103 exists on the conductive electrode layer 102 where electric currents are flowing. The reaction layer 106 can be formed by carrying out electroplating.

The surface of the reaction layer 106 may be treated with organic molecules or specific chemical materials that selectively react with a specific substance. The surface treatment of the reaction layer 106 can be selectively performed by connecting the electrical source with the electrode unit 300 that is connected with the conductive electrode layer 102 disposed under the reaction layer 106 whose surface will be treated and flowing electric currents.

When forming the reaction layer 106 and treating the surface of the reaction layer 106, in order to minimize cross contamination that may occur between substances forming the reaction layer 106 or chemical substances formed on the surface of the reaction layer 106, starting from a process where materials with the lowest reactivity are used, overall processes may be carried out in order.

As shown in (f) and (g) of fig. 7, the method for forming a biosensor may further include a step of transferring a structure where the upper insulation layer is formed onto a silicone patch 300 by the use of a PDMS stamp 500 before step S160 of doping the conductive substance at the graphene layer 103 exposed by the opening part formed in the upper insulation layer of the sensing unit 100.

The reference electrode can be formed by transferring the structure where the upper insulation layer 105 is formed to the silicon patch 600, providing an electrical source to the electrode unit 300 connected with the conductive electrode layer 102 disposed under a specific graphene layer 103 of a plurality of graphene layers 103 exposed by the opening part 105a to flow electric currents in the conductive electrode layer 102 of the sensing unit 100, and carrying out electroplating to deposite Ag/AgCl. Herein, the specific graphene layer 103 means the graphene layer on which the reaction layer 106 will be formed. Subsequently, the humidity sensor can be formed by depositing PEDOT (poly(3,4-ethylenedioxythiophene)) in the same way as described above. For example, in the humidity sensor, first and second electrodes may be comb-shaped, and a groove of the first electrode and a groove of the second electrode may cross each other. After using polyaniline to form the pH sensor, the glucose sensor can be formed by treating prussian blue, glucose oxidase and Nafion® in order. Herein, the prussian blue functions as a catalyst in the breakdown of peroxide that is the product of the breakdown of glucose, and the glucose oxidase is a breakdown enzyme of glucose. As above, the biosensor having the humidity sensor, the pH sensor and the glucose sensor in one platform can be formed.

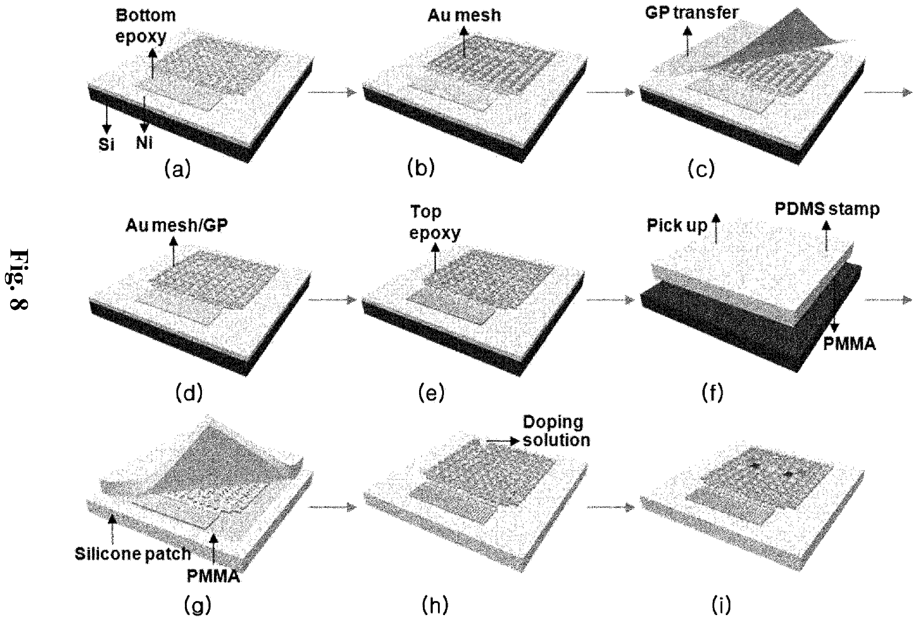

Fig. 8 schematically shows a process of forming a biosensor according to an embodiment of the present invention.

Referring to Fig. 8, a nickel layer (Ni) falling under the sacrifice layer is formed by depositing a nickel metal on the silicon substrate (Si). The lower insulation layer (Bottom epoxy) is formed by carrying out a spin coating of epoxy resin on the nickel layer (Ni) and carrying out photolithography.

After carrying out thermal evaporation to deposit chrome (Cr) 7nm and gold (Au) 70nm, the conductive electrode layer (Au mesh) is formed by carrying out photolithography. In the sensing unit and the bridge unit, the conductive electrode layer (Au mesh) may be formed in net patterns or mesh patterns.

The graphene is transferred onto the conductive electrode layer (Au mesh). The graphene layer (GP) is formed by patterning the graphene in the same shape as the conductive electrode layer.

The upper insulation layer (Top epoxy) having the same patterns as the lower insulation layer (Bottom epoxy) is formed by carrying out a spin coating of epoxy resin on the graphene layer (GP) and carrying out photolithography. At this time, the upper insulation layer of the sensing unit and the electrode unit is removed so that the opening part is formed. The structure is transferred onto the silicone patch (Silicone patch) using the PDMS stamp.

Au nanoparticles are doped at the graphene layer of the sensing unit by carrying out a drop casting of a solution of 20mM AuCl₃ to the graphene layer of the sensing unit exposed by the opening part and producing a reaction for about 5 minutes.

The reaction layer belonging to each sensing unit is formed and the surface of each reaction layer is treated so that various sensors are formed in one platform.

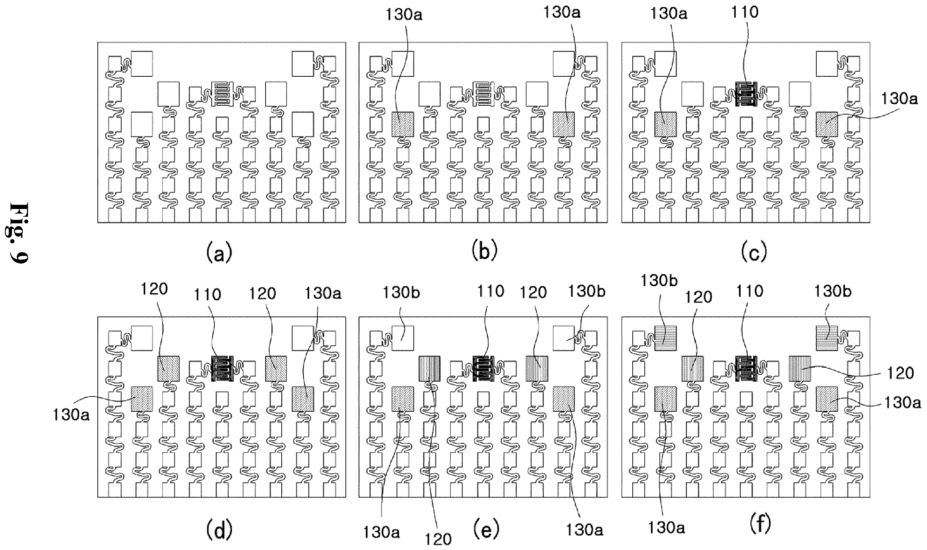

Fig. 9 is a view for explaining a process of treating the surface of a biosensor according to an embodiment of the present invention. In this biosensor, in order to detect humidity, acidity, glucose and the like, a plurality of reaction layers including working electrode, reference electrode and the like may be formed on the graphene layer where gold nanoparticles are doped. At this time, in order to minimize cross contamination, a reaction layer consisting of materials with the lowest reactivity may be formed in the first place.

Referring to Fig. 9, by transferring the structure with the upper insulation layer formed thereon to the silicone patch (See (a) of Fig. 9), flowing electric currents in the conductive electrode layer of the sensing unit by providing an electronic source to the electrode unit connected with the conductive electrode layer disposed under the graphene layer where the reaction layer 106 will be formed, and carrying out electroplating to deposit silver/silver chloride (Ag/AgCl), the reference electrode 130a is formed (See (b) of Fig. 9).

In the same way as described above, the humidity sensing unit 110 is formed by depositing PEDOT (poly(3,4-ethylenedioxythiophene)) (See (c) of Fig. 9). At this time, in the humidity sensing unit 110, first and second electrodes may be comb-shaped, and grooves of the first and second electrodes may cross each other.

Subsequently, the pH sensing unit 120 is formed using polyaniline (See (d) of Fig. 9), and the glucose sensing unit 130b are formed by treating prussian blue, glucose oxidase and Nafion® in order (See (e) ∼ (f) of Fig. 9). Herein, the prussian blue functions as a catalyst in the breakdown of peroxide that is the product of the breakdown of glucose, and the glucose oxidase is a breakdown enzyme of glucose. The reference electrode 130a of the glucose sensing unit also functions as a reference electrode of the pH sensing unit 120. By this, the biosensor with the humidity sensor, the pH sensor and the glucose sensor in one platform can be formed.

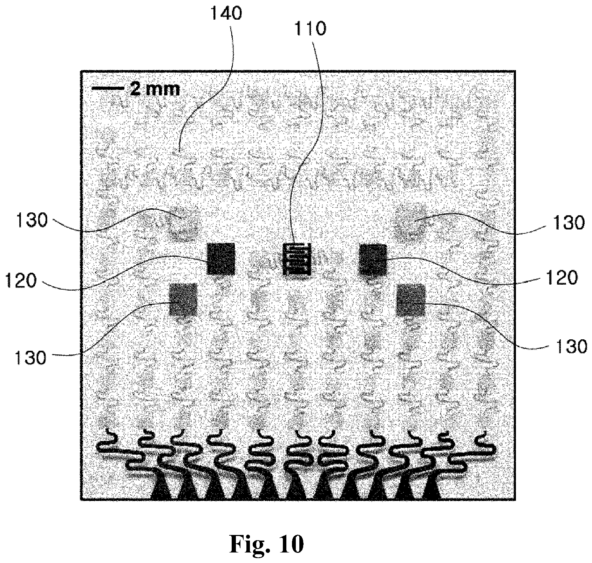

Fig. 10 is an image of a biosensor formed according to a embodiment of the present invention.

Referring to Fig. 10, in the biosensor, the conductive electrode with mesh patterns and the graphene layer with gold doped thereat function as a working electrode, and there is an active layer formed of prussian blue on the graphene layer. Moreover, the active layer is encapsulated with glucose oxidase and a substance called as Nafion.

If there is glucose in sweat, the glucose oxidase makes a reaction to generate peroxide, electrons are generated owing to the prussian blue's function as a catalyst in the breakdown of peroxide, and the working electrode captures the electrons. That is, it is possible to electrically measure the change of glucose in sweat.

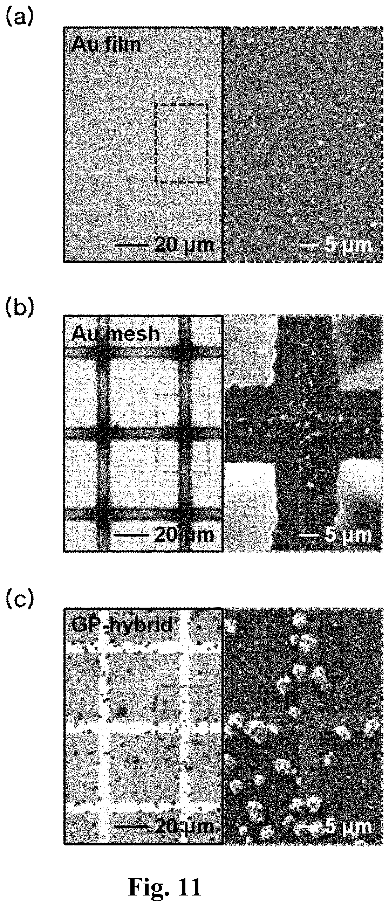

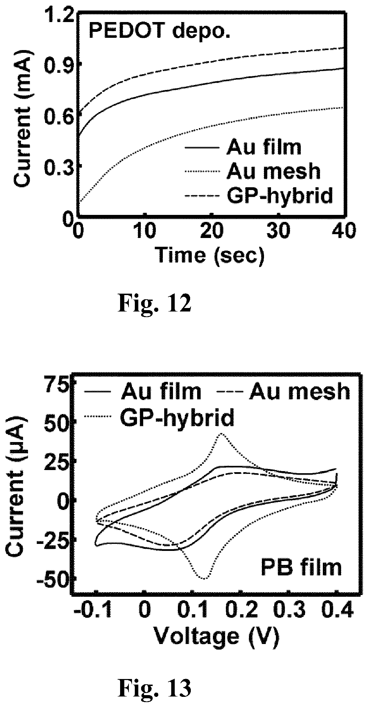

Fig. 11 shows deposition results of polyethylenedioxythiophene depending on electrode structures composing a biosensor according to an embodiment of the present invention, and Fig. 12 is graphs analyzing the feature of electrodes related to deposition results of polyethylenedioxythiophene depending on electrode structures composing a biosensor according to an embodiment of the present invention.

Referring to Figs. 11 and 12, in order to analyze an electrode feature of the biosensor, an electrode consisting of a gold film only (Au film), a gold electrode with mesh patterns (Au mesh), and a graphene hybrid electrode according to an embodiment of the present invention that includes a gold electrode with mesh patterns and a graphene layer with gold nanoparticles doped thereat (GP hybrid) are compared and analyzed. Especially, when comparing and analyzing the deposition result of PEDOT for measuring the humidity, in case of the electrode of the gold film, PEDOT is deposited on the entire electrode since electric currents flow in the entire area of the electrode. In case of the gold electrode with mesh patterns, there are areas associated with the mesh patterns where PEDOT was not deposited. In case of the hybrid electrode of the present invention, PEDOT is deposited on the entire electrode.

Fig. 13 is graphs analyzing the electrochemical feature depending on electrode structures composing a biosensor according to an embodiment of the present invention.

Referring to Fig. 13, the electrochemical feature of the graphene hybrid electrode formed according to an embodiment of the present invention is superior to that of the electrode of the gold film, and the surface treatment is also improved in the graphene hybrid electrode of the present invention. This is due to the result that the surface feature of the electrode is improved by the graphene. In addition, the electrochemical activity of the graphene hybrid electrode formed according to an embodiment of the present invention is higher. Herein, the electrochemical activity means how many electrons subjects to be measured can give and take at the same area.

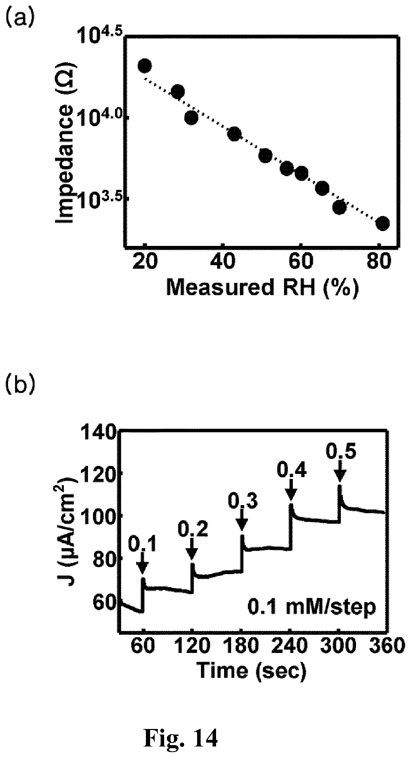

Fig. 14 is graphs analyzing operational features of a biosensor according to an embodiment of the present invention.

As shown in Fig. 14, the biosensor shows a very stable performance as a sensor even though the humidity sensor, the glucose sensor and the pH sensor are formed in one platform.

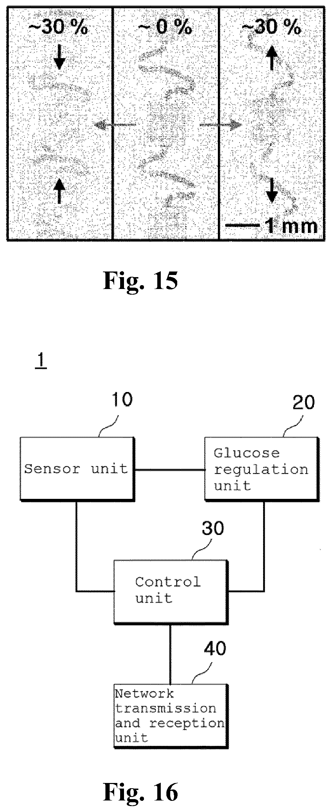

Fig. 15 shows the stretchability of a biosensor according to an embodiment of the present invention.

Referring to Fig. 15, the biosensor formed according to an embodiment of the present invention can have excellent stretchability due to the bridge unit with the multiply-bent shape. Accordingly, when testing the sensor's performance under the contracted or expanded state by about 30%, there is no falloff in the biosensor's feature. Since the biosensor according to an embodiment of the present invention is very flexible and has excellent stretchability, it can be easily adjusted to wearable devices.

Fig. 16 shows the constitution of a glucose control system according to an embodiment of the present invention.

Referring to Fig. 16, the glucose control system 1 may include a sensor unit 10, a glucose regulation unit 20, a control unit 30 and a network transmission and reception unit 40. Since the sensor unit 10 is the same as above described biosensor, the repeated explanation is not made here.

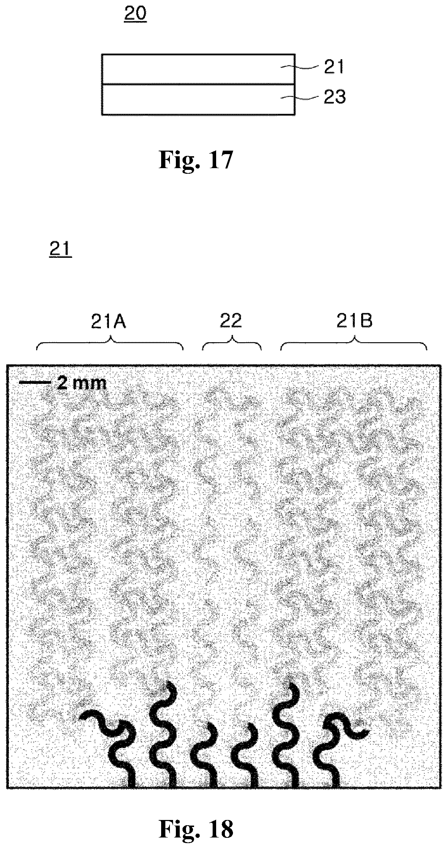

Fig. 17 shows a regulation unit of a glucose control system according to an embodiment of the present invention.

Referring to Fig. 17, the glucose regulation unit 20 may include a heating part 21 and a drug delivery part 23 disposed under the heating part 21.

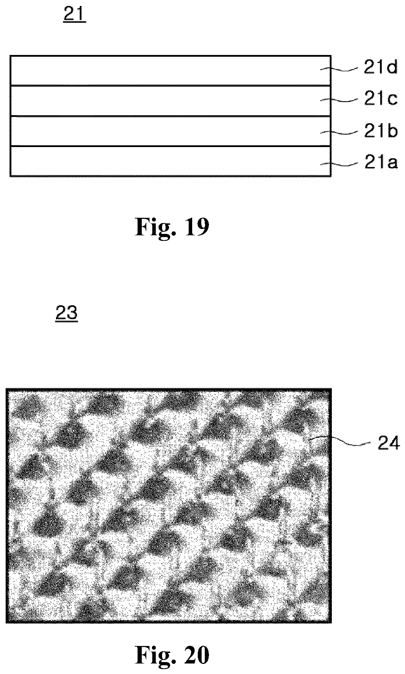

Fig. 18 is an image of a heating part according to an embodiment of the present invention, and Fig. 19 is a sectional view of a heating part of a glucose control system according to an embodiment of the present invention.

Referring to Figs. 18 and 19, the heating part 21 may include a first heating part 21A, a second heating part 21B and a temperature sensor 22. The first and second heating parts 21A, 21B can regulate a dose of a drug injected into a skin by stages. The temperature sensor 22 can measure and regulate the temperature of the first and second heating parts 21A, 21B so that the temperature of the first and second heating parts 21A, 21B does not rise beyond a predetermined value.

The first and second heating parts 21A, 21B may include net or mesh patterns. The first and second heating parts 21A, 21B have a plurality of horizontal linear patterns formed in a horizontal direction and a plurality of vertical linear patterns formed in a vertical direction. There are intersecting points formed by crossing the horizontal linear patterns and vertical linear patterns. Horizontal linear pattern or vertical linear pattern between each intersecting point may multiply bent in the up/down or left/right direction and include the intersecting point. Therefore, the length of the horizontal linear pattern or vertical linear pattern may be longer than the length between each intersecting point. As above, the horizontal linear patterns or vertical linear patterns are multiply bent and longer than the length between each intersecting point so that the heating part 21 can exhibit an excellent heating effect.

The heating part 21 may include a lower insulation layer 21a, a conductive electrode layer 21b, a graphene layer 21c and an upper insulation layer 21d that are accumulated in order.

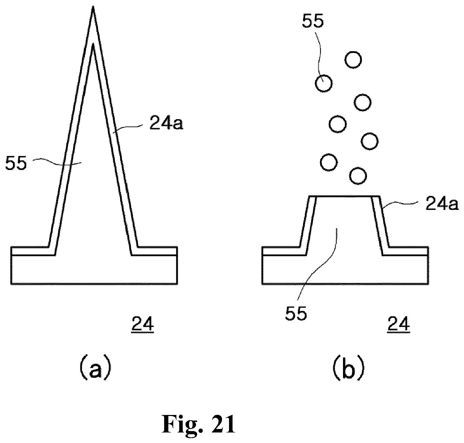

Fig. 20 shows a drug delivery part in a glucose control system according to an embodiment of the present invention, and Fig. 21 shows a fine needle of a drug delivery part in a glucose control system according to an embodiment of the present invention.

Referring to Figs. 20 and 21, the drug delivery part may include micro-sized fine needles 24 that are spaced apart from each other in a predetermined distance. The fine needle 24 may be formed of a high molecular substance, for example vinyl pyrrolidone. The fine needle 24 may include a coating layer 24a formed of a phase change material (PCM) on its exterior face, and contain a glucose regulation drug 55 therein. The phase change material's phase is changed at predetermined temperature or more. For example, the phase change material may be tridecanoic acid. If temperature becomes critical temperature (T_{c}) or more, a phase change to a liquid state occurs at the coating layer 24a of the fine needle 24 so that the glucose regulation drug 55 inside the fine needle 24 flows out to permeate into a skin.

Even though not shown in drawings, the glucose control system 1 may further include an electric source device that provides an electric source to the sensor unit 10 and the heating part 21. The electric source device can provide the electric power to the sensor unit 10 and the heating part 21 using an electric power cable, a rechargeable battery or a disposable battery. If the electric power is provided to the sensor unit 10 and/or the heating part 21 of the glucose control system 1 by the electric source device, electric currents flow in the sensor unit 10 and/or the heating part 21.

Referring again to Figs. 2 and 16, the control unit 30 receives signals from the sensors 11, 12, 13, 14 included in the sensor unit 10. The control unit 30 receives humidity signals from the humidity sensor 11 to measure the humidity. The control unit 30 receives signals from the glucose sensor to measure the glucose concentration in sweat of the user when the humidity is equal to or more than a predetermined value. The control unit 30 receives signals from the pH sensor 12 to measure the pH value. The control unit 30 receives signals from the strain gauge 14 to measure the strain. The control unit 30 amends the measured glucose concentration. In an enzyme based electrochemical sensor, if the pH becomes low, signals could be distorted and thus measurement errors could occur. The control unit 30 can amend the measured glucose concentration based on the measured pH value. Signals could be also distorted by the humidity change resulting from the amount of sweat, the strain change resulting from the movement, and the like. The control unit 30 can amend the glucose concentration more precisely based on the measured humidity and strain along with the measured pH value. When the glucose concentration of the user is high, the control unit 30 makes electric currents flow in the heating part 21 of the glucose regulation unit 20 to increase the temperature of the heating part 21. By this, when the temperature is a predetermined value or more, the phase change of the high molecular substance of the fine needle 24 of the drug delivery part 23 occurs and thus the glucose regulation drug 55 inside the fine needle 24 is injected into the skin. If the injected amount of the drug is too much, user's hand tremors are measured through the strain gauge 14 included in the sensor unit 10 and a blood sugar state is diagnosed as a low blood sugar state.

The network transmission and reception unit 40 can receive the diagnosed result from the control unit 30 to send it to the user's wireless terminal, the user's family, certain hospital or a service provider interacting with the glucose control system 1. The network means a connection structure that can exchange data between wire-wireless terminals and servers, and can be realized as a wire-wireless communication network such as a local area network (LAN), a wide area network (WAN), a value added network (VAN), a mobile radio communication network, a satellite communication network, etc.

The wireless terminal may include a portable terminal and/or a computer. The portable terminal is a wireless communication device guaranteeing portability and mobility, and may include PCS (personal communication system), GSM (global system for mobile communications), PDC (personal digital cellular), PHS (personal handyphone system), PDA (personal digital assistant), IMT-2000 (international mobile telecommunication-2000), CDMA-2000 (code division multiple access-2000), W-CDMA (W-code division multiple access), WiBro (wireless broadband internet) terminal, smart phone, smart pad, etc. The computer may include desktop, laptop, tablet PC, and the like with WEB browser installed therein.

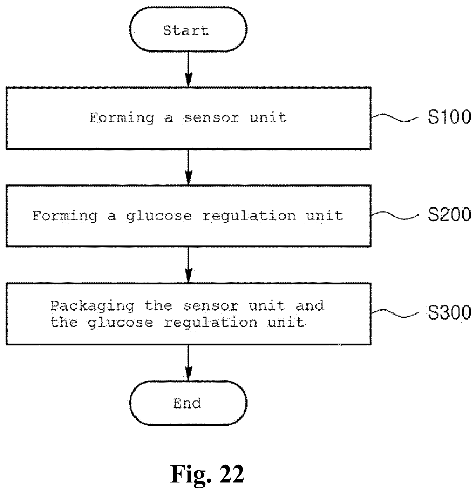

Fig. 22 is a flowchart for explaining a method for forming a glucose control system according to an embodiment of the present invention.

Referring to Fig. 22, the method for forming a glucose control system includes step S100 of forming a sensor unit, step S200 of forming a glucose regulation unit and step S300 of packaging the sensor unit and the glucose regulation unit. The forming order may be changed. Since the step of forming a sensor unit was explained in the embodiment previously described with reference to Fig. 6, the detailed explanation is not made here.

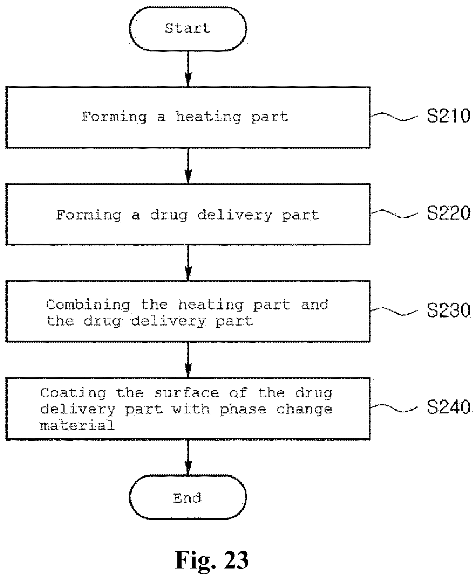

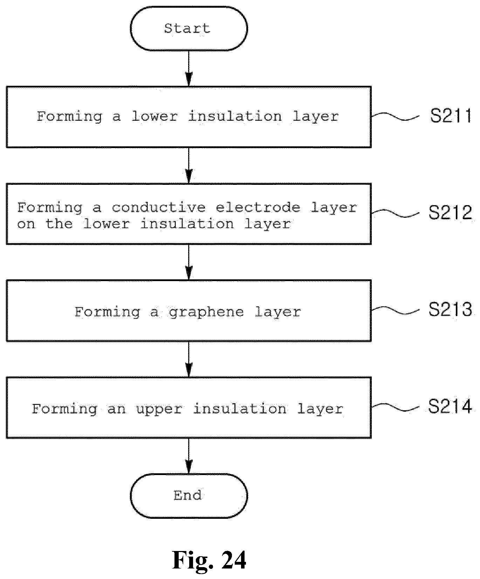

Fig. 23 is a flowchart for explaining a method for forming a glucose regulation unit according to an embodiment of the present invention, and Fig. 24 is a flowchart for explaining a method for forming a heating part of a glucose regulation unit according to an embodiment of the present invention.

Referring to Figs. 23 and 24, step S200 of forming a glucose regulation unit includes step S210 of forming a heating part, step 220 of forming a drug delivery part, step S230 of combining the heating part and the drug delivery part, and step S240 of coating the surface of the drug delivery part with phase change material.

Step S210 of forming a heating part includes step S211 of forming a lower insulation layer, step S212 of forming a conductive electrode layer on the lower insulation layer, step S213 of forming a graphene layer and step S214 of forming an upper insulation layer.

Referring again to Figs. 18, 19, 23 and 24, in step S210 of forming a heating part, a sacrifice substrate may be used in order to make the process easy. Glass substrate, quartz substrate, silicone substrate, germanium substrate and the like may be used as the sacrifice substrate. A sacrifice layer for separating the glucose regulation unit from the substrate may be formed on the sacrifice substrate. For example, the sacrifice layer may be formed of metals such as Ni, Cu, Al or PMMA (poly(methyl methacrylate), etc.

In step S211, the lower insulation layer 21a is formed on the sacrifice substrate. The lower insulation layer 21a can be formed by carrying out a spin coating of a nonconductive high molecular substance on the sacrifice substrate where the sacrifice layer is formed. The high molecular substance may include photosensitive polymer. For example, the high molecular substance may include epoxy resin, polyimide, parylene, etc. For example, the lower insulation layer 21a can be formed by coating the photosensitive polymer substance on the sacrifice substrate and carrying out photolithography or e-beam lithography process. By this, at the lower insulation layer 21a, there may be a plurality of horizontal linear patterns extending in a horizontal direction, a plurality of vertical linear patterns extending in a vertical direction and intersecting points located at sites where the horizontal linear patterns and vertical linear patterns cross each other.

In step S212, a conductive electrode layer 21b is formed on the lower insulation layer 21a. The conductive electrode layer 21b can be formed by depositing a conductive substance on the lower insulation layer 21a and carrying out photolithography or e-beam lithography process to pattern it in the same shape as the lower insulation layer 21a. The conductive electrode layer 21b may be formed in a net shape or a mesh shape in order to decrease the entire resistance of the heating part 21.

In step S213, a graphene layer 21c is formed on the conductive electrode layer 21b. The graphene layer 21c can be formed by transferring graphene grown by chemical vapor deposition onto the conductive electrode layer 21b and patterning the graphene in the same shape as the conductive electrode layer 21b. The graphene layer may be formed in a single layer or in two or more layers, and may be grown directly on the conductive electrode layer 21b.

In step S214, an upper insulation layer 21d is formed on the graphene layer 21c. The upper insulation layer 21d can be formed by coating a nonconductive photosensitive high molecular substance on the graphene layer 21c and patterning the coated substance in the same shape as the lower insulation layer 21a.

When forming the conductive electrode layer 21b and/or the graphene layer 21c, the temperature sensor 22 can be formed together between the first and second heating parts 21A, 21B. The temperature sensor 22 may include only the graphene layer 21c without the conductive electrode layer 21b.

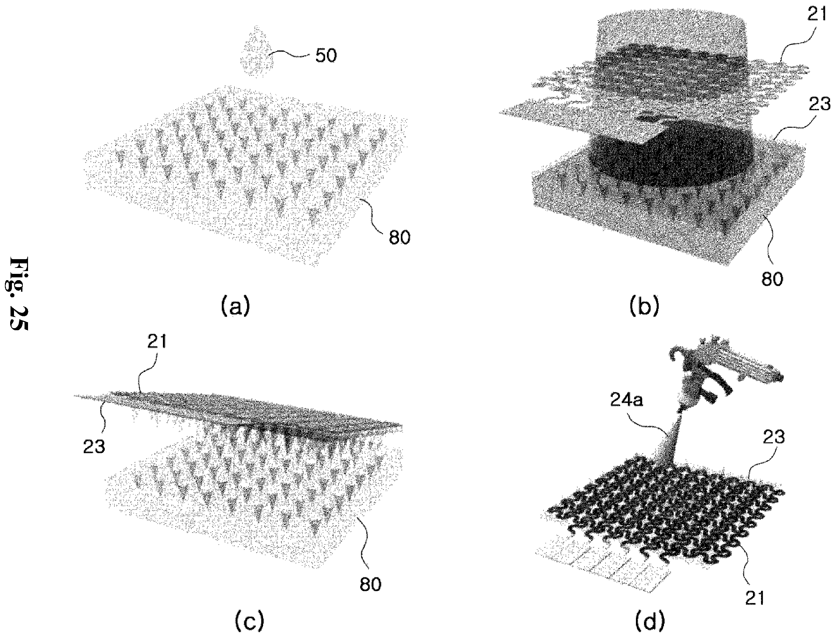

Fig. 25 is a view for explaining a formation of a drug delivery part and a method of combining a drug delivery part and a heating part according to an embodiment of the present invention.

Referring to Figs. 23 and 25, in step S220, the drug delivery part 23 is formed by a mold 80. A process for forming the drug delivery part 23 can be simplified without any complicated step by using the mold 80. The drug delivery part 23 where micro-sized fine needles are spaced apart from each other in a predetermined distance can be formed by pouring a mixed solution 50 of a high molecular substance and a drug into the mold 80 and hardening it. The mold 80 has a concave portion corresponding to the fine needle. The concave portion may have about 250µm diameter and about 1mm height as an example. The mixed solution 50 may include a commercialized blood sugar depressing agent, a high molecule, a hardening agent, etc. For example, the mixed solution 50 may comprise metformin, vinyl pyrrolidone and azobisisobutyronitrile.

In step S230, the drug delivery part 23 and the heating part 21 are combined. The heating part 21 can be combined at one side of the drug delivery part 23 by disposing the heating part 21 at one side of the drug delivery part 23, carrying out a dry process within a vacuum chamber of room temperature and shedding UV light for about 30 minutes. The structure where the heating part 21 and the drug delivery part 23 are combined is separated from the mold 80.

In step S240, the phase change material is coated on the surface of the fine needle of the drug delivery part 23. The surface of the fine needle 24 can be coated with the phase change material 24a by the process such as spray coating, dip coating, drop casting, etc. For example, the phase change material may be tridecanoic acid.

Referring again to Fig. 22, in step S300, the sensor unit 10 and the glucose regulation unit 20 are packaged in one patch. The patch may be formed of a transparent high molecular substance with excellent adhesive property on skin. The sensor unit 10 and the glucose regulation unit 20 may be encircled by a transparent patch with excellent adhesive property on skin. The patch may include separate films that can control sweat. As a result, the glucose control system can minimize water evaporation by the patch.

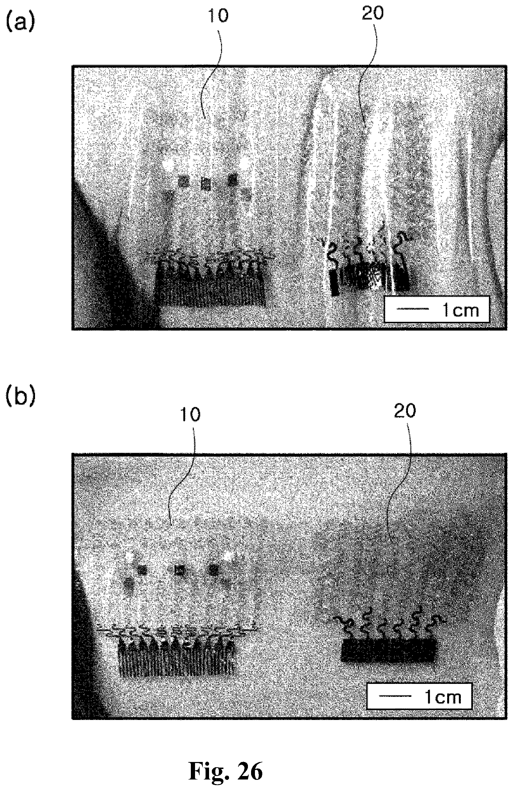

Fig. 26 is an image of a sensor unit and a glucose regulation unit formed according to an embodiment of the present invention.

Referring to Fig. 26, the sensor unit 10 and the glucose regulation unit 20 of the glucose control system can be packaged into one by a transparent patch, and are flexible and have excellent stretchability.

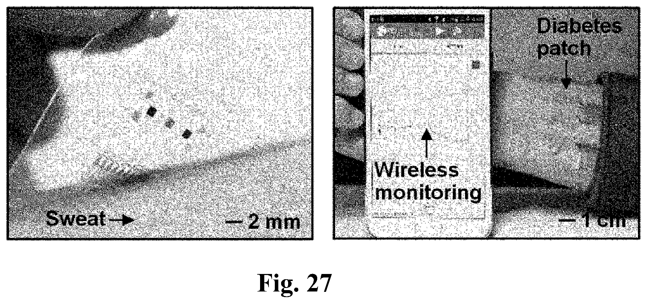

Fig. 27 is an image of a glucose control system formed according to an embodiment of the present invention.

Referring to Fig. 27, the sensor unit and the glucose regulation unit are formed on one patch in the glucose system, and the glucose control system can be attached to a skin. The control unit of the glucose control system can diagnose a blood sugar state as a low blood sugar state or a high blood sugar state based on the glucose concentration in the body of the user, and wirelessly send the diagnosed result to the user's terminal interacting with the glucose control system. By this, the user can control the glucose concentration in his or her own body in real time.

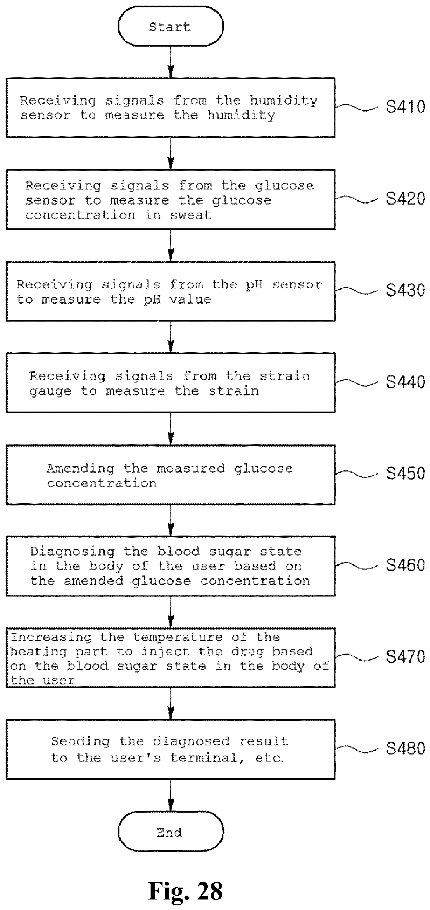

Fig. 28 is a flowchart for explaining a method for controlling glucose using a glucose control system according to an embodiment of the present invention.

Referring to Fig. 28, the method for controlling glucose may include step S410 of receiving signals from the humidity sensor of the sensor unit to measure the humidity, step S420 of receiving signals from the glucose sensor to measure the glucose concentration in sweat, step S430 of receiving signals from the pH sensor to measure the pH value, step S440 of receiving signals from the strain gauge to measure the strain, step S450 of amending the measured glucose concentration, step S460 of diagnosing the blood sugar state in the body of the user based on the amended glucose concentration, step S470 of increasing the temperature of the heating part to inject the drug based on the blood sugar state in the body of the user and step S480 of sending the diagnosed result to the user's terminal, etc.

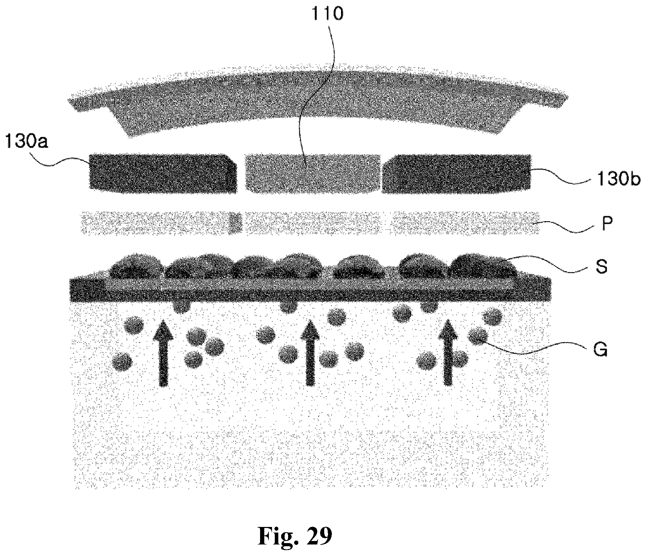

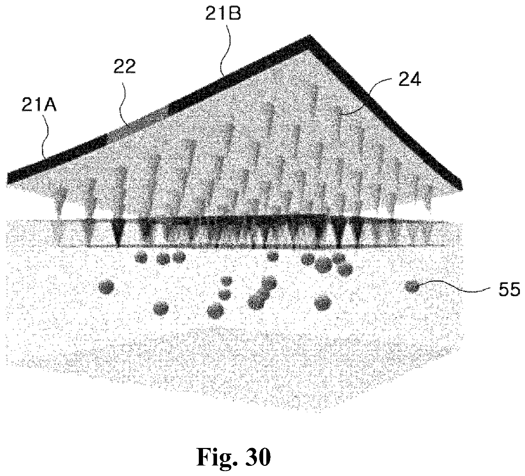

Fig. 29 is a view for explaining an operation of a sensor unit in a glucose control system according to an embodiment of the present invention, and Fig. 30 is a view for explaining an operation of a glucose regulation unit in a glucose control system according to an embodiment of the present invention.

Referring again to Figs. 2, 16, 28, 29 and 30, the glucose control system 1 may include the sensor unit 10 for measuring the glucose concentration and the glucose regulation unit 20 for regulating the glucose concentration in the body in one package. The sensor unit 10 may include the humidity sensor 11, the pH sensor 12, the glucose sensor 13 and the strain gauge 14.

If the glucose control system is attached to the skin, sweat is captured by a sweat capturing layer (P) existing in the patch of the glucose control system. In step S410, the control unit 30 receives signals from the humidity sensor 11 to measure the humidity in order to check whether the captured sweat reaches a certain level before analyzing the glucose concentration in the body.

In step S420, when the measured humidity is equal to or more than the certain level, the control unit 30 receives signals from sensing units 130a and 130b of the glucose sensor 13 to measure the glucose concentration in sweat. The glucose sensor 13 is an electrochemical based sensor, and a conductive electrode layer with mesh patterns and a graphene layer with gold doped thereon operate as a working electrode. An active layer that is formed of prussian blue is disposed on the graphene layer, and the active layer may be encapsulated with glucose oxidase and a substance called as Nafion.

If there is glucose (G) in sweat (S), the glucose oxidase makes a reaction to generate peroxide, electrons are generated owing to the prussian blue's function as a catalyst in the breakdown of peroxide, and the working electrode captures the electrons. The control unit 30 can receive signals from the glucose sensor 13 to measure the glucose concentration in sweat when the humidity is equal to or more than a predetermined value.

In step S430, the control unit 30 receives signals from the pH sensor 12 to measure the pH value. The pH change resulting from lactic acid included in sweat can be measured.

In step S440, the control unit 30 receives signals from the strain gauge 14 to measure the strain. The strain change resulting from a factor such as movement and the like can be measured.

In step S450, the control unit 30 amends the measured glucose concentration. In an enzyme based electrochemical sensor, if the pH becomes low, signals could be distorted and thus measurement errors could occur. The control unit 30 can amend the measured glucose concentration based on the measured pH value. Signals could be also distorted by the humidity change resulting from the amount of sweat, the strain change resulting from the movement and the like. The control unit 30 can amend the glucose concentration more precisely based on the measured humidity and strain along with the measured pH value.

In step S460, the control unit 30 diagnoses a blood sugar state in the body as a low blood sugar state or a high blood sugar state based on the measured glucose concentration.

In step S470, the control unit 30 increases the temperature of the heating parts 21A, 21B to inject a drug based on the diagnosed blood sugar state in the body of the user. When the user's state is diagnosed as a high blood sugar state, the control unit 30 increases the temperature of the heating parts 21A, 21B by providing the electrical source to the glucose regulation unit 20 to make electrical currents flow in the heating parts 21A, 21B. When the temperature of the heating parts 21A, 21B increase to reach about 41 ∼ 42°C, the drug delivery part 43 is heated by the heating parts 21A, 21B so that the phase change of the phase change material coated at the surface of the fine needle 24 occurs. As a result, the glucose regulation drug 55 encircled by the phase change material permeates into the skin and the glucose concentration in the body can be controlled.

The heating parts 21A, 21B are divided into a first heating part 21A and a second heating part 21B, and thus the amount of the drug injected into the skin can be regulated by stages. For example, the control unit 30 can periodically diagnose the user's state, and increase the temperature of the first heating part 21A to inject the drug included in the fine needle 24 of the drug delivery part 23 disposed under the first heating part 21A into the user when the user's state is diagnosed as the high blood sugar state at a first period. After that, when the user's state is diagnosed as the high blood sugar state at a second period, the control unit 30 can increase the temperature of the second heating part 21B to inject the drug included in the fine needle 24 of the drug delivery part 23 disposed under the second heating part 21B into the user. The drug delivery part 23 may be replaceable. The glucose control system 1 can be used consistently by replacing the drug delivery part 23.

In order for the temperature of the first and second heating parts 21A, 21B not to rise to a predetermined value or more, the amount of the electric currents flowing in the first and second heating parts 21A, 21B can be controlled by disposing the temperature sensor 22 between the first and second heating parts 21A, 21B to measure temperature in real time.

In addition, the control unit 30 can measure the user's movement in real time to diagnose the user's state as a low blood sugar state when there are user's hand tremors.

In step S480, the control unit 30 sends the diagnosed state of the user to the user's wireless terminal, the user's family, certain hospital or a service provider interacting with the glucose control system through the network transmission and reception unit 40.

The control unit 30 can be materialized as a record medium containing commands executable in a computer, for example, a program module executed by a computer. The computer readable medium may be any usable medium that is accessible by the computer, and include volatile and non-volat6ile medium and/or separation type and non-separation type medium. The computer readable medium may include computer storage medium and communication medium.

Although the embodiments of the present invention have been disclosed for illustrative purposes, those skilled in the art will appreciate that the present invention may be embodied in other specific ways without changing the technical spirit or essential features thereof. Therefore, the embodiments disclosed in the present invention are not restrictive but are illustrative. The scope of the present invention is given by the claims, rather than the specification, and also contains all modifications within the meaning and range equivalent to the claims.

### Industrial Applicability

A biosensor according to embodiments of the present invention can have excellent reliability since various features such as the feature of the interface are improved. The biosensor can be easily and variously applied to a wearable apparatus that is used in the state of being attached to the body since it can have excellent flexibility and stretchability. The biosensor can include various sensors on one platform so that various substances can be detected simultaneously. In the biosensor, it is not necessary to prepare a reference electrode separately since reference electrode and working electrode can be formed together in one platform. The biosensor may include one or more sensors, and the one or more sensors may be easily formed by a simple process.

The glucose control system according to embodiments of the present invention can measure glucose concentration in a noninvasive manner. The glucose control system can accurately measure glucose concentration in the user's body by amending the measured glucose concentration with reference to pH value, humidity, strain, etc. The glucose control system can control the glucose concentration in the user's body while measuring the glucose concentration in real time. The glucose control system can have excellent flexibility and stretchability so that it can be easily used in the state of being attached to a body.

## Claims

1. A biosensor comprising at least one sensor comprising:
a sensing unit;
a bridge unit connected to the sensing unit; and
an electrode unit connected to the bridge unit,
wherein the sensing unit comprises a graphene layer.

2. The biosensor of claim 1, wherein the graphene layer comprises a doped conductive substance.

3. The biosensor of claim 2, wherein the conductive substance comprises at least one selected from metal nanoparticles and metal nanowires.

4. The biosensor of claim 1, wherein the sensing unit further comprises a conductive electrode layer disposed under the graphene layer and a reaction layer disposed on the graphene layer.

5. The biosensor of claim 4, wherein the conductive electrode layer comprises net patterns or mesh patterns.

6. The biosensor of claim 4, wherein the sensing unit further comprises an upper insulation layer disposed on the graphene layer, the upper insulation layer has an opening part exposing the graphene layer, and the reaction layer is in contact with the graphene layer through the opening part.

7. The biosensor of claim 4, wherein the reaction layer is formed of different substances depending on a type of the sensor.

8. The biosensor of claim 4, wherein the reaction layer is formed of silver/silver chloride or PEDOT (poly(3,4-ethylenedioxythiophene)).

9. The biosensor of claim 4, wherein a surface of the reaction layer is treated by at least one substance selected from polyaniline, prussian blue and glucose oxidase.

10. The biosensor of claim 1, wherein the bridge unit has a multiply-bent shape.

11. The biosensor of claim 1, wherein the sensor comprises at least one sensor selected from a humidity sensor, a pH sensor, a glucose sensor and a strain gauge.

12. The biosensor of claim 1, further comprising:
an electric source unit providing an electric source to the electrode unit, and
a processing unit that receives any one signal of electric current, voltage or impedance from the electrode unit to convert the signal.

13. A method for forming a biosensor that comprises at least one sensor comprising a sensing unit, a bridge unit connected to the sensing unit and an electrode unit connected to the bridge unit, comprising:
forming a lower insulation layer;
forming a conductive electrode layer on the lower insulation layer;
forming a graphene layer on the conductive electrode layer; and
forming a reaction layer on the graphene layer.

14. The method for forming a biosensor of claim 13, further comprising:
forming an upper insulation layer on the graphene layer, the upper insulation layer having an opening part that exposes the graphene layer, and
doping a conductive substance at the graphene layer through the opening part before forming the reaction layer.

15. The method for forming a biosensor of claim 14, wherein the conductive substance comprises at least one selected from metal nanoparticles and metal nanowires.

16. The method for forming a biosensor of claim 14, wherein the reaction layer is formed on the graphene layer through the opening part.

17. The method for forming a biosensor of claim 13, wherein the conductive electrode layer comprises net patterns or mesh patterns.

18. The method for forming a biosensor of claim 13, wherein the conductive electrode layer is formed of a substance comprising at least one selected from gold, aluminum, platinum, nickel, graphene, silver nanowire film, metal grid and indium tin oxide.

19. The method for forming a biosensor of claim 13, wherein the reaction layer is formed of different substances depending on a type of the sensor.

20. The method for forming a biosensor of claim 13, wherein the sensing unit, the bridge unit and the electrode unit are formed together by a same process.

21. The method for forming a biosensor of claim 13, wherein the sensor comprises at least one sensor selected from a humidity sensor, a pH sensor, a glucose sensor and a strain gauge.

22. A glucose control system comprising:
a sensor unit comprising a glucose sensor;
a glucose regulation unit regulating glucose concentration in a body of a user; and
a control unit controlling the sensor unit and the glucose regulation unit.

23. The glucose control system of claim 22, wherein the sensor unit further comprises at least one selected from a humidity sensor, a pH sensor and a strain gauge.

24. The glucose control system of claim 23, wherein the control unit receives signals from the glucose sensor to measure glucose concentration in sweat of the user, receives signals from the pH sensor to measure pH value, and amends the measured glucose concentration based on the pH value.

25. The glucose control system of claim 24, wherein the control unit receives signals from the humidity sensor to measure humidity, receives signals from the strain gauge to measure strain, and amends the measured glucose concentration based on the pH value, the humidity and the strain.

26. The glucose control system of claim 24, wherein the control unit receives signals from the humidity sensor to measure humidity, and receives signals from the glucose sensor to measure the glucose concentration in sweat of the user when the humidity is equal to or more than a predetermined value.

27. The glucose control system of claim 24, wherein the control unit diagnoses a blood sugar state in the body of the user based on the amended glucose concentration.

28. The glucose control system of claim 27, wherein the control unit controls the glucose regulation unit in order to inject a glucose regulation drug into the user based on the diagnosed blood sugar state in the body of the user.

29. The glucose control system of claim 23, wherein the control unit receives signals from the strain gauge to measure strain, and diagnoses a blood sugar state in the body of the user as a low blood sugar state based on the strain.

30. The glucose control system of claim 22, wherein the glucose regulation unit comprises a drug delivery part that comprises a fine needle comprising a glucose regulation drug and a heating part that is disposed on the drug delivery part and heats the drug delivery part to increase a temperature of the drug delivery part, a surface of the drug delivery part is coated with a phase change material whose phase is changed at critical temperature or more, and the glucose regulation drug is released from the fine needle by heating the heating part.

31. The glucose control system of claim 30, wherein the drug delivery part injects the glucose regulation drug through the user's skin by the fine needle, and the glucose regulation drug comprises blood sugar depressing agents.

32. The glucose control system of claim 30, wherein the heating part comprises a first heating part, a second heating part adjacent to the first heating part, and a temperature sensor that is disposed between the first and second heating parts and measures temperature of the first and second heating parts, and further wherein the control unit controls temperature of the first and second heating parts when the temperature measured by the temperature sensor is equal to or more than a predetermined temperature.

33. The glucose control system of claim 30, wherein the heating part comprises multiply-bent patterns.

34. The glucose control system of claim 22, wherein the sensor unit comprises a sensing unit, a bridge unit connected to the sensing unit and an electrode unit connected to the bridge unit, and the sensing unit comprises a graphene layer.

35. The glucose control system of claim 34, wherein the graphene layer comprises a doped conductive substance, and the conductive substance comprises at least one selected from metal nanoparticles and metal nanowires.

36. The glucose control system of claim 34, wherein the sensing unit further comprises a conductive electrode layer disposed under the graphene layer and a reaction layer disposed on the graphene layer, the conductive electrode layer comprises net patterns or mesh patterns, and the reaction layer is formed of different substances depending on a type of the sensing unit.

37. The glucose control system of claim 34, wherein the bridge unit has a multiply-bent shape.

38. The glucose control system of claim 22, further comprising a network transmission and reception unit that sends the blood sugar state in the body of the user diagnosed by the control unit to a user terminal interacting with the glucose control system.

39. A method for forming a glucose control system comprising:
forming a sensor unit comprising a glucose sensor;
forming a glucose regulation unit; and
packaging the sensor unit and the glucose regulation unit.

40. The method for forming a glucose control system of claim 39, wherein the step of forming a glucose regulation unit comprises
forming a heating part,
forming a drug delivery part that comprises a fine needle comprising a glucose drug,
combining the heating part and the drug delivery part, and
coating the surface of the drug delivery part with a phase change material.

41. The method for forming a glucose control system of claim 39, wherein the sensor unit comprises at least one sensor comprising a sensing unit, a bridge unit connected to the sensing unit and an electrode unit connected to the bridge unit, and the step of forming the sensor unit comprises
forming a lower insulation layer,
forming a conductive electrode layer on the lower insulation layer,
forming a graphene layer on the conductive electrode layer, and
forming a reaction layer on the graphene layer.

42. The method for forming a glucose control system of claim 41, further comprising
forming an upper insulation layer on the graphene layer, the upper insulation layer having an opening part that exposes the graphene layer, and
doping a conductive substance at the graphene layer through the opening part before forming the reaction layer, the conductive substance comprising at least one selected from metal nanoparticles and metal nanowires.

43. The method for forming a glucose control system of claim 42, wherein the reaction layer is formed on the graphene layer through the opening part, and the reaction layer is formed of different substances depending on a type of the sensor.

44. A method for controlling glucose using a glucose control system that comprises a sensor unit comprising a glucose sensor and a pH sensor, a glucose regulation unit regulating glucose concentration in a body of a user, and a control unit controlling the sensor unit and the glucose regulation unit, comprising:
receiving signals from the glucose sensor to measure glucose concentration in sweat of the user;
receiving signals from the pH sensor to measure pH value; and
amending the measured glucose concentration based on the pH value.

45. The method for controlling glucose of claim 44, wherein the sensor unit further comprises at least one selected from a humidity sensor and a strain gauge, the method further comprising receiving signals from the humidity sensor to measure humidity and receiving signals from the strain gauge to measure strain, and further wherein the measured glucose concentration is amended based on the pH value, the humidity and the strain.

46. The method for controlling glucose of claim 45, further comprising diagnosing a blood sugar state in the body of the user based on the amended glucose concentration.

47. The method for controlling glucose of claim 46, further comprising injecting a glucose regulation drug into the user by the glucose regulation unit based on the diagnosed blood sugar state in the body of the user.

48. The method for controlling glucose of claim 44, wherein the sensor unit further comprises a humidity sensor, the method further comprising receiving signals from the humidity sensor to measure humidity, and further wherein signals are received from the glucose sensor and the glucose concentration in sweat of the user is measured when the humidity is equal to or more than a predetermined value.

49. The method for controlling glucose of claim 44, further comprising receiving signals from the strain gauge to measure strain, wherein a blood sugar state in the body of the user is diagnosed as a low blood sugar state based on the strain.
